# EUROPEAN PATENT APPLICATION

(11) **EP 1 344 830 A1**
(43) Date of publication of application: **17.09.2003**
(21) Application number: 02075989.0
(22) Date of filing: 12.03.2002
(51) Int. Cl.: C12P 35/02, C12P 35/06, C12P 39/00, C12N 9/08, C12N 9/80, C12N 9/06, C12N 11/02, C12N 11/18, C07D 501/20, C07D 501/04

(54) **A process for the preparation of cephalosporan acid derivatives from cephalosporin C**

(71) Applicant: Bioferma Murcia, S.A., 30840 Alhama de Murcia, Murcia (ES)
(72) Inventor: Guisan Seijas, Jose M., 28002 Madrid (ES); Fernandez-Lafuente, Roberto, 28914 Madrid (ES); Fernandez-Lorente, Gloria, 28030 Madrid (ES); Betancort, Lorena Dutrenit, 28028 Madrid (ES); Hidalgo, Aurelio Huertas, 48013 Bilbao (ES); Mateo, Cesar Gonzalez, 28005 Madrid (ES)
(74) Representative: Davila Baz, Angel

(57) **Abstract**

A process for the preparation of cephalosporin acid derivatives comprises the step of enzymatically converting cephalosporin C into 7 cephalosporin acid derivatives in the presence of two biocatalysts, wherein one biocatalyst comprises co-immobilised D-amino acid oxidase and a catalase enzyme, and the other biocatalyst comprises immobilised glutaryl acylase.

## Description

### Introduction

7 amino cephalosporanic acid (7ACA) is a key intermediate in the synthesis of semi-synthetic cephalosporins which are pharmaceutically important β-lactam antibiotics.

7 ACA is currently obtained, typically, by a chemical route that requires several protection-deprotection steps, very low temperatures and the use of chlorinated solvents. Substitution by a biocatalytic route is advantageous and solves the problem of an environmentally undesirable process and also avoids tedious chemical protection/deprotection reactions. Such a biocatalytic route based on penicillin G or V acylase for the production of 6 aminopenicillanis acid (6APA) and 7-amino-deacetoxi-cephalosporanic acid (7ADCA) is known. However there is no cephalosporin C acylase equivalent suitable for use in an industrial process.

One alternative enzymatic route for the production of 7 ACA is based on the action of two different enzymes, D-amino acid oxidase (DAAO) and glutaryl acylase. This biocatalytic approach has been attempted using whole cells, however this may have undesirable side effects such as contamination of the product and undesirable side reactions catalyzed by other enzymes. The use of cell-free enzymes also requires a complex two-reactor enzymatic process each involving a different enzyme.

The first process is an oxidative deamination of cephalosporin C catalysed by DAAO, to yield α-keto-adipyl-7ACA with the production of hydrogen peroxide as a side product. Hydrogen peroxide promotes a rapid inactivation of the immobilised enzymes as well as promoting the oxidative decarboxylation of α-ketoadipyl-7ACA into glutaryl-7ACA. The industrial objective in this first step is to obtain the maximum yield of glutaryl-7ACA, because the remaining α-ketoadipyl-7ACA is considered not to be hydrolysed by the second enzyme (glutaryl acylase). To achieve this, additional hydrogen peroxide is routinely added after the full oxidation of cephalosporin. This may be added in the same reaction vessel or in a different reaction vessel [1 to 5].

In a further reactor, 7ACA is produced by hydrolysis of glutaryl-7ACA by the enzyme glutaryl acylase.

The enzymes DAAO and glutaryl acylase cannot be used in the same reactor because of the very deleterious effect of hydrogen peroxide on the stability of both DAAO and glutaryl acylase. In particular glutaryl acylase is very sensitive to hydrogen peroxide and glutaryl acylase is typically used in a second reactor to avoid the deleterious effect of the hydrogen peroxide produced.

The main drawbacks of the current enzymatic methodologies are as follows:
- DAAOs are very unstable because of inactivation by hydrogen peroxide;
- In order to achieve almost 100% conversion of cephalosporin C to glutaryl-7ACA additional hydrogen peroxide is required. This is both time consuming and requires an additional reactor vessel;
- It is not possible to carry the reaction out in a single reactor because of the sensitivity of glutaryl acylase to hydrogen peroxide;
- DAAO biocatalyst are not designed for improved thermal stability which is indicative of the long term stability under industrial conditions; and
- Under conventional conditions, the main stability problem of the DAAO biocatalyst is the inactivation by hydrogen peroxide.

There is a need therefore for an improved process for the preparation of 7ACA from cephalosporin C on a large factory scale, which will address at least some of these problems.

### Statements of invention

According to the invention there is provided a process for the preparation of cephalosporin acid derivatives comprising the step of enzymatically converting cephalosporin C into 7 cephalosporin acid derivatives in the presence of two biocatalysts, wherein one biocatalyst comprises co-immobilised D-amino acid oxidase and a catalase enzyme, and the other biocatalyst comprises immobilised glutaryl acylase.

Preferably the reaction is carried out with the co-immobilised DAAO and catalase and the immobilised glutaryl acylase present in a single reactor.

In one embodiment of the invention the reaction may be carried out in a single basket reactor or other reactor type wherein the co-immobilised DAAO and catalase are physically separated from the immobilised glutaryl acylase.

In one embodiment of the invention the three enzymes are present in one biocatalyst system comprising co-immobilised DAAO, catalase and glutaryl acylase enzymes.

Preferably the or each biocatalyst system is immobilised on highly activated aldehyde or epoxy supports, most preferably highly activated glutaraldehyde or glyoxyl supports.

In one embodiment of the invention the D-amino acid oxidase and catalase enzymes are co-immobilised on highly activated glyoxyl or epoxy supports.

Preferably the supports have large internal surfaces to permit intense multipoint and multi-subunit covalent attachment. The supports may be selected from any one or more of agarose gels, silica porous beads, porous glass, epoxy acrylic resins or cellulose.

In one embodiment of the invention the D-amino acid oxidase is obtained from one or more of *Rhodotorula gracilis* or *Trigonopsis variabilis*. The catalase enzyme is preferably obtained from one or more of mammalian liver, *Thermus sp, Bacillus sterothermophillus, Thermotoga sp, Micrococcus lysodelikus, Aspergillus niger* and glutaryl acylase is preferably obtained from *Acinetobacter sp*.

The invention provides Cephalosporin acid derivatives whenever produced by a process of the invention.

The invention also provides ketoadipyl 7-ACA whenever produced by a process of the invention.

### Brief Description of Drawings

The invention will be more clearly understood from the following description thereof given by way of example only with reference to the accompanying drawings, in which:-
Fig. 1 is a graph showing the thermal stability (measured by the percentage of residual activity) of two DAAO derivatives prepared on different supports. The triangles represent a derivative prepared on glutaraldehyde-agarose. The squares represent a derivative prepared on glyoxyl-agarose;
Fig. 2 is a graph showing the difference in thermal stability of immobilised enzyme from *T. variabilis* (DAO-T) and immobilised enzyme from *R. gracilis* (DAO-R);
Fig. 3 is a graph showing the thermal stability of different catalases immobilised on agarose activated with glutaraldehyde. The squares represent *Micrococcus lysodeikticus* catalase, the circles represent catalase from bovine liver and the triangles represent catalase from *Aspergillus oryzae;*
Fig. 4 is a graph showing the thermal stability of different immobilised derivatives of catalase from *Micrococcus lysodeikticus*. The rhombus represents soluble enzyme, the circles represent derivative immobilised on glyoxyl agarose, the squares represent derivative immobilised on glutaraldehyde-agarose and the triangles represent derivative absorbed on PEI coated supports;
Fig. 5 is a graph showing the thermal stability of DAAO/catalase immobilised derivative. The circles represent DAAO activity and the triangles represent catalase activity;
Fig. 6 is a graph showing the synthesis of α-keto-adipyl 7ACA from Ceph C catalysed by co-immobilised DAAO and catalase represented as percentage yield over time;
Fig. 7 is a graph showing the hydrolysis of α-keto-adipyl-7ACA by glutaryl acylase shown as percentage yield over time. The triangles represent pure α-ketoadipyl-7ACA and the circles represent α-ketoadipyl-7ACA plus glutaric acid;
Fig. 8 is a graph showing the hydrolysis of α-ketoadipyl-7ACA by glutaryl acylase where a higher pH of pH8 is used in the last step of the reaction. The results are shown as percentage yield versus time. The triangles represent a pH fixed at 7.5 and the circles represent the final increment of pH8; and
Fig. 9 is a graph showing the synthesis of 7ACA from Ceph C catalysed by two biocatalysts co-immobilised DAAO and catalase and glutaryl acylase in one reactor. The results are shown as percentage yield versus time.

### Detailed description

The invention provides a novel route for the production of 7ACA from cephalosporin C using two different biocatalysts comprising three different enzymes in a single reactor. This novel route overcomes the problems encountered with the currently used enzymatic route. In addition, the conventional two reactor process is greatly improved by the *in situ* decomposition of hydrogen peroxide.

The first biocatalyst comprises D-amino acid oxidase (DAAO) and a catalase co-immobilised on the same support particle. By using this biocatalyst, hydrogen peroxide, a product of the reaction catalyzed by DAAO, is instantaneously decomposed by the catalase into water and oxygen. Co-immobilisation of catalase with DAAO thereby reduces the hydrogen peroxide in the environment where DAAO is placed for example in the pores of the support material. In addition the time-lag before the decomposition of hydrogen peroxide is significantly reduced and the normal decarboxylation of α-ketoadipyl-7ACA is avoided [6].

In this way, the enzymes present in the reactor are not exposed to the very deleterious effects of high concentrations of hydrogen peroxide. Moreover, the oxidation of cephalosporin C can be stopped when pure α-ketoadipyl 7ACA is formed without the formation of glutaryl 7ACA which is typically formed using conventional routes.

The second biocatalyst comprises immobilised glutaryl acylase, which has been found to exhibit a significant catalytic activity on the hydrolysis of α-ketoadipyl 7ACA mainly in the absence of glutaryl 7ACA or glutaric acid. The presence of an excess of either glutaryl 7ACA or glutaric acid results in a strong inhibition of the hydrolysis of α-ketoadipyl 7ACA catalysed by glutaryl acylase. For this reason, under conventional conditions where more than 85% of cephalosporin C is transformed into glutaryl 7ACA and only 15-5% into α-ketoadipyl 7ACA, the α-ketoadipyl 7ACA is not hydrolysed by glutaryl acylase. Therefore only if most of the cephalosporin C is transformed into α-ketoadipyl 7ACA is there good activity of the glutaryl acylase against the α-ketoadipyl 7ACA.

It is also possible to have a single biocatalyst comprising three enzymes. This has the advantage of improved kinetics. However, the disadvantage is that when one of the enzymes is inactivated all three need to be discarded. In the present invention the two biocatalysts may be physically separate. A basket reactor may be used wherein one of the biocatalysts (for example the physically softer derivative) may be placed inside the basket and the other biocatalyst is located outside the basket. This allows a single biocatalyst to be discarded when it becomes inactivated or less stable. In addition it is possible to replace one of the enzymes when it loses its activity.

In comparison with the conventional two-step enzymatic process where a catalase enzyme is not present, the invention has the following technological advantages.
- D-amino acid oxidases can be used in the absence of hydrogen peroxide. The oxidases are stable and therefore the biocatalyst is operationally more stable. In the absence of hydrogen peroxide, glutaryl acylase can be used with DAAO in the same reactor.
- In the absence of glutaryl 7ACA and glutaric acid, the enzyme glutaryl acylase exhibits good catalytic activity towards α-ketoadipyl 7ACA with no significant inhibitory effects during the course of the hydrolytic reaction.
- As a result of the improved stability of the biocatalysts the invention allows the process of producing 7 ACA to be carried out in one step and using only one reactor.
- The process also provides isolated α-ketoadipyl-7ACA which can be further modified to produce semi-synthetic cephalosporin derivatives.

The process of the invention involves enzymes and a co-immobilisation process which are designed to produce biocatalysts with a high thermal stability. High thermal stability ensures the long term stability of the biocatalyst.

The immobilisation of enzymes inside the porous support also increases the enzyme stability by preventing any intermolecular process (proteolysis, aggregation) and by preventing the enzyme from interactions with external interfaces such as air, oxygen or immiscible organic solvents. However, random immobilisation does not promote any additional conformational stabilisation of immobilised enzymes. In fact, there are reports of immobilisation procedures with no effect or even negative effects on the stability of some enzymes. However, it is generally accepted that the stabilisation of the enzymes should be achieved if the immobilisation of each enzyme molecule occurs through several residues, mainly if the reactive groups in the support are secluded from its surface through short spacer arms. In this way, all the residues of the enzyme molecule involved in immobilisation have to preserve their relative positions almost completely unaffected during any conformational change promoted by heat, organic solvents or any other distorting agent. Therefore multipoint covalently immobilised enzymes are in general more stable than their soluble counterparts or randomly immobilised derivatives.

The reaction between two structures as different and complex as a protein and a support surface is very complex. In the invention highly activated preexisting solids are used not only to immobilise proteins, but to achieve multipoint covalent attachment between the immobilised enzyme and the support. Epoxy or aldehyde (e.g. glyoxyl) groups have excellent properties which give stabilising multi-point immobilisations that are summarised as follows:
i.- they can be attached to the support surface through short spacer arms;
ii.- a very dense monolayer of reactive and stable epoxy or aldehyde groups can be easily prepared;
iii.- epoxy-groups are able to react with groups in proteins that are very abundant in the protein surface (amino, thiol, phenolic, imidazole), aldehyde reacts with primary amino groups usually exposed in the surface of the protein;
iv.- the reaction between the nucleophile groups in proteins with epoxy or aldehyde groups on the support is hardly affected by steric hindrances;
v.-chemical modification of the enzyme is minimal;
v.- enzyme-support reaction is easily stopped by blocking the epoxy groups with different reagents such as mercaptoethanol, ethanolamine or glycine, or by reducing the aldehyde and the shift bases; and
vi.- linear aldehydes have an additional advantage. One point attachment is very weak and reversible. Typically, the enzyme is immobilised via, at least, the simultaneous promotion of, at least, two bonds between the enzyme and the support. Therefore, the enzyme is immobilised via the area rich in primary amino groups (not via the most reactive amine). These areas of the enzyme (where there is a high density of reactive groups) are where an intense multipoint covalent attachment is more likely.

A strong "intramolecular" attachment is achieved between the already immobilised enzyme and the highly activated supports via short spacer arms. However, such multipoint covalent immobilisation requires more drastic experimental conditions than those necessary to covalently immobilise the proteins (perhaps via a single very reactive residue) in order to enhance the reactivity of its surface nucleophilic groups. The promotion of strong multipoint immobilisation requires enhancement of the reactivity of the enzyme residues placed in the neighbourhood of the support surface. Such reactivity is very low at neutral pH, conditions where standard immobilisation protocols are carried out. For example, surface lysine residues, the most common nucleophilic group on the enzyme surface, preserves a fairly intact pK around 10.5 and hence they are hardly reactive at neutral pH. In addition, the reactive groups on the enzyme and the reactive groups on the support may not be correctly aligned - in fact proteins and solid supports are very dissimilar in structure. As a result the formation of strong multipoint covalent immobilisation requires very long periods of time. In addition, the use of moderately high temperatures (e.g. 25°C) promotes a lower rigidity on the enzyme molecule and favours its reactivity towards the dense layer of reactive groups on the support. In fact, such variables (pH, time and temperature) have been previously found to greatly increase the formation of multipoint covalent linkages between many enzymes and glyoxyl-agarose gels or epoxy supports [7 to 13].

Multimeric enzymes constitute a specific problem. It is important to involve all the subunits in the immobilisation rather than establishing a strong multipoint attachment involving only one of the subunits. The general properties of the system are similar to achieving multipoint covalent attachment, however the orientation of the enzyme should cover the area where there are more subunits involved. Further crosslinking with polyfunctional macromolecules involves the subunits not directly immobilised on the support [14].

To improve the enzyme stability, supports and activation protocols that enable the establishment of a strong multipoint covalent attachment between the enzyme and the support are used. Supports that offer large surfaces highly activated with reactive groups are suitable for strong multipoint covalent attachment and conditions which support enzyme-multi-interaction is favoured. Such supports may be selected from any one or more of silica, porous glass, agarose, epoxy acrylic matrices or cellulose. The reactive groups may be selected from any one or more of aldehydes such as glyoxyl or epoxy groups.

Biodiversity is a powerful tool in improving the properties of a biocatalyst. Enzyme stability, activity, possibility for stabilisation via immobilisation and enzyme size need to be considered in selecting the optimal enzyme. The enzyme size determines the pore size of the support and consequently the maximum loading capacity of the support.

To improve the first biocatalyst different amino acid oxidases (from *Rhodotorula gracilis* or *Trigonopsis variabilis*) and different catalases (from *Aspergillus niger, Micrococcus lysodeikticus,* bovine liver and from some thermophilic microorganisms) were immobilised on different supports activated with different reactive groups such as epoxide, glutaraldehyde or glyoxyl group. In all cases, the stabilisation of the enzymes by multipoint and multisubunit immobilisation was assessed.

The most stable derivatives of the D-amino acid oxidase were obtained when using the enzyme from *Rhodotorula gracilis* using multipoint and multisubunit covalent immobilisation on highly activated glyoxyl supports (e.g. glyoxyl-agarose gels). The enzyme is very unstable in free form because of the rapid dissociation of the subunits, compared to the soluble enzyme from *Trigonopsis*. After multisubunit and multipoint immobilisation, the stability of *R*. *gracilis* was better than that of derivatives from *T. variabilis* where loss of cofactor was a major problem. With *R. gracilis* the co-factor remains very tightly bound to the enzyme and the first step of inactivation is the dissociation of the cofactor. This may be prevented via multisubunit immobilisation and, therefore, after stabilisation of the quaternary structure, this enzyme immobilisation may be considered the most suitable one for carrying the process out.

The catalases also exhibit improved thermal stability when immobilised on activated glyoxyl supports. The best results in terms of stability were achieved with catalases from *Micrococcus lysodeikticus* (from commercial catalases) and from thermophilic microorganisms from *Thermus sp. Bacillus sterothermophillus* or *Thermotoga sp.* In this way, co-immobilised derivatives of both enzymes on the same support could be prepared. These derivatives exhibit a very high thermal stability: a half-life time of 1 day for each enzyme when inactivated at 60^{º}C. When inactivated at 25^{º}C, the half-life is, in both cases, higher than 100 days.

The stability of the second biocatalyst was also improved by multipoint covalent immobilisation of glutaryl acylase from *Acinetobacter* sp. on glutaraldehyde-activated or glyoxyl-activated supports.

All three enzymes may be stabilised/immobilised via immobilisation on aldehyde or epoxy supports. Therefore, if just one biocatalyst is desired, co-immobilisation of the three enzymes may be achieved.

The invention provides a single-reactor enzymatic process for the transformation of cephalosporin C into 7ACA by combining the following features:
a.- the preparation of very stable enzyme derivatives - stable native enzymes are further stabilised by multipoint covalent immobilisation on highly activated supports;
b.- the instantaneous elimination of hydrogen peroxide, by co-immobilisation of catalase and D-amino acid oxidase on the same support; and
c.- the prevention of the undesirable formation of glutaryl-7ACA and glutaric acid that have an inhibitory effect on the hydrolysis of α-keto adipyl-7ACA by glutaryl acylase.

The invention will be more clearly understood from the following examples.

### Example 1: Preparation of DAAO derivatives

2000 IU of DAAO (from *T. variabilis* or *R. gracilis*) was added to 90 ml of 100 mM sodium bicarbonate pH 10 T 22^{º}C, containing 7 g of 10% crosslinked glyoxyl agarose activated with 210 µmol aldehyde/ml, under continuous stirring. Full immobilisation is achieved after a few minutes, but the support enzyme multi-interaction is left to proceed for 3 h. 100 mg of sodium borohydride was added as a reaction end point. After 30 minutes, derivatives were washed with an excess of water. Activity recovered was over 70%.

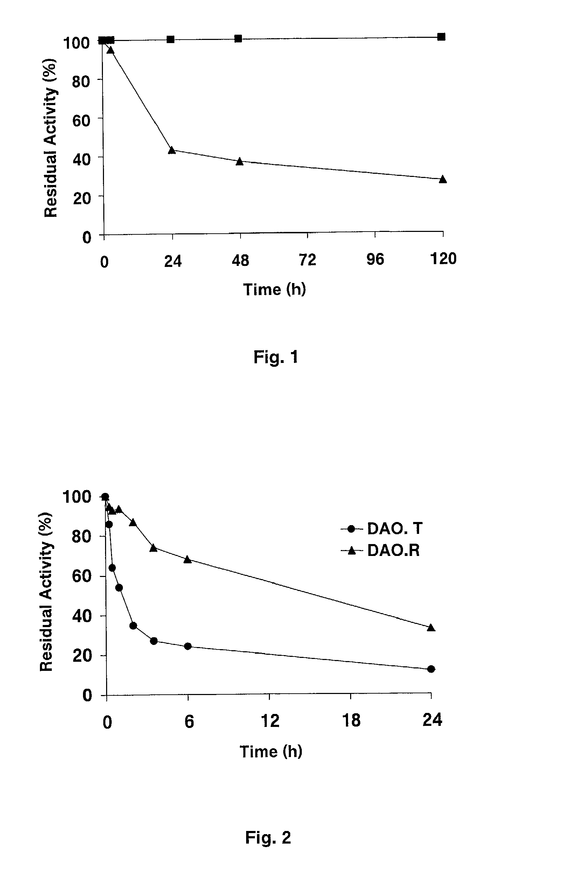

It was found that optimal derivatives presented their quaternary structure stabilised, and that promoted a spectacular improvement in *R. gracilis* stability (that mainly was inactivated via dissociation of the subunits) while *T. variabilis* enzyme was not so stabilised because the main cause of enzyme inactivation was the dissociation of the cofactor (that could not be prevented via immobilisation). In fact, multipoint covalent attachment of the DAAO from *R. gracilis* allowed further stabilisation of the enzyme (it was found to be about 20,000 fold more stable than soluble enzyme). These derivatives were also more stable than others prepared in poorly activated glyoxyl supports or glutaraldehyde supports as shown in Fig. 1.

Immobilised derivatives were incubated in 0.1M potassium phosphate at pH7.5 at 45°C. At different times aliquots were withdrawn and assayed under standard conditions.

Inactivation of the different enzymes on highly activated glyoxyl supports showed that while soluble DAAO from *T. variabilis* was more stable than DAAO from *R. gracilis,* the immobilised enzymes exhibited an opposite behavior, the enzyme from *R. gracilis* being much more stable than one isolated from *T. variabilis* as shown in Fig. 2, where the optimal derivatives of both enzymes where incubated at 60°C at pH 7.5.

### Example 2: Preparation of catalase derivatives

300 mg of catalase (e.g., from *Micrococcus lysodeikticus*) was added to 90 mL of 100 mM sodium bicarbonate pH 10 at 22°C, containing 7 g of 6% crosslinked (e.g., glyoxyl agarose activated with 75 µmol aldehyde/ml,) under continuous stirring. Full immobilisation is achieved after a few minutes, but the support enzyme multi-interaction was left to proceed for 3 h. 100 mg of sodium borohydride was added as a reaction end point. After 30 minutes, derivatives were washed with an excess of water. Activity recovery was over 60%.

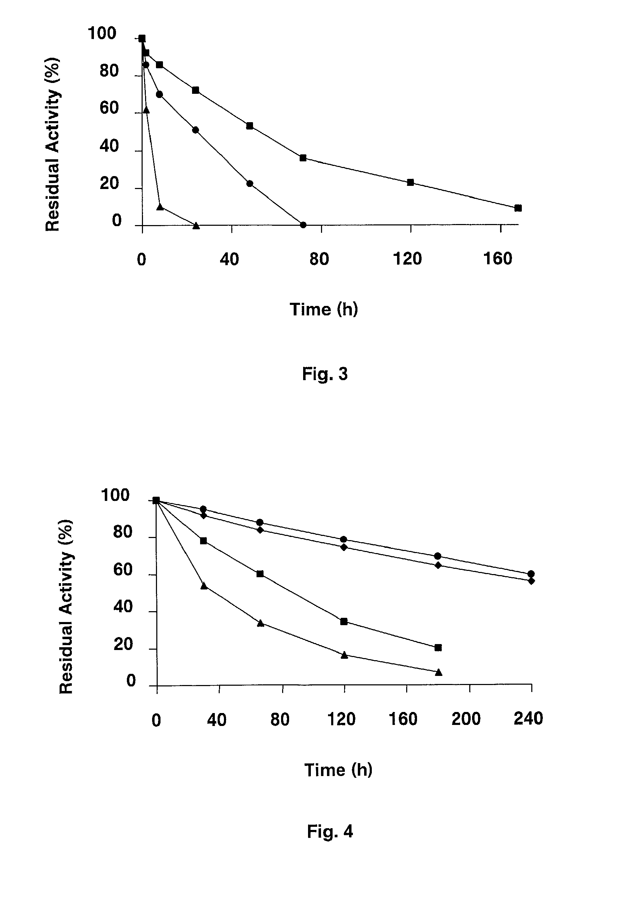

Among the commercially available catalases, the one from *Micrococcus lysodeikticus* exhibited the best stability properties (Fig. 3), both in free or immobilised form. Inactivation was carried out at 25°C and at pH 7.5. Glyoxyl derivatives were found to be more stable than other derivatives as shown in Fig. 4 (inactivations in this case were performed at 60°C).

Immobilised thermophilic catalases were found to be much more stable even than the best derivative from mesophilic miroorganisms, suggesting better prospects for their use as industrial catalysts. For example, the enzyme from *Thermus* immobilised in a glutaraldehyde support remained fully active after one month incubation at 45°C at pH 7.

### Example 3: Preparation of co-immobilised DAAO-catalase derivatives

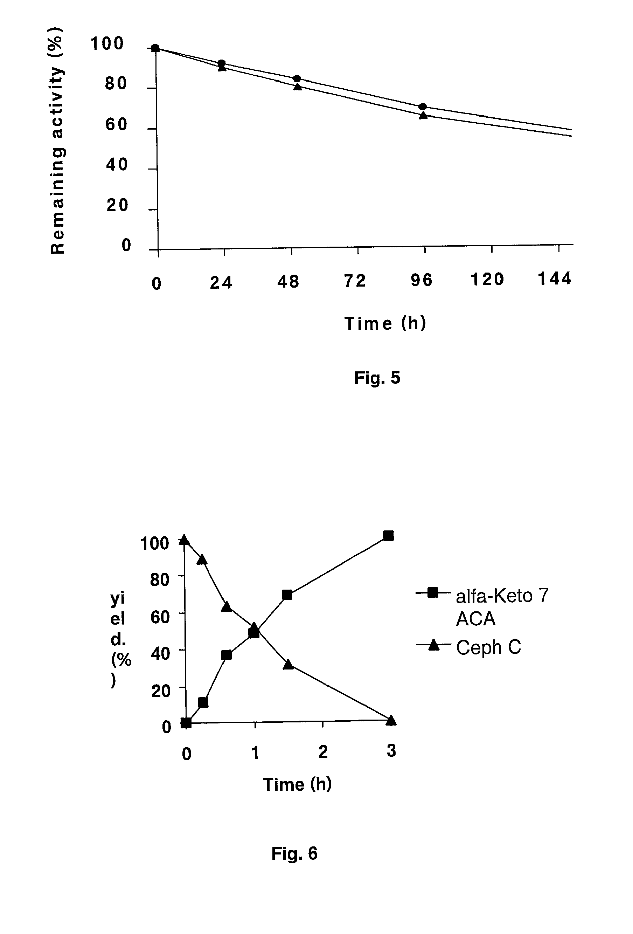

50 mg DAAO (e.g., from *R. gracilis*) and 250 mg of catalse (e.g., from bovine liver or *M. lysodeikticus*) was added to a suspension of 90 ml of 0.1 M sodium, bicarbonate buffer at pH 10 and 20°C and submitted to very gently stirring. After a few minutes, the enzymes are immobilised, but the enzyme-support reaction was left to continue for 3 h. 100 mg of sodium borohydride was added as a reaction end point. After 30 minutes, derivatives were washed with an excess of water. The molecular properties (activity/stability) for the individual enzymes (DAAO and catalase) correspond exactly to the individual derivatives described in examples 1 and 2, and are very similar to each other as shown in Fig. 5 where the coimmobilised derivative was incubated in 50mM phosphate buffer at pH7.5 and 45°C.

### Example 4: Production of α-ketoadipyl-7ACA by DAAO co-immobilised with catalase

100 ml of Cephalosporin C (e.g., 25 mM) was dissolved at pH 7.5 in 100 mM potassium phosphate at 22°C, then 1 g of co-immobilised derivative as described in example 3 was added to the reaction mixture under bubbling of oxygen. No production of glutaryl 7-ACA was detected. After the reaction, HPLC analysis showed only a peak that could be associated to pure α-keto adipyl 7 ACA. The reaction course is shown in Fig. 6.

### Example 5: Production of 7ACA from α-ketoadipyl 7-ACA using glutaryl acylase

Samples of pure α-ketoadipyl 7-ACA and glutaryl 7 ACA were hydrolysed at pH 7.5 and 22°C by using commercial immobilised glutaryl acylase (supplied by Roche). The activity was 33 micromoles/min/g for glutaryl acylase and of 5.3 micromoles/min/g, a good activity.

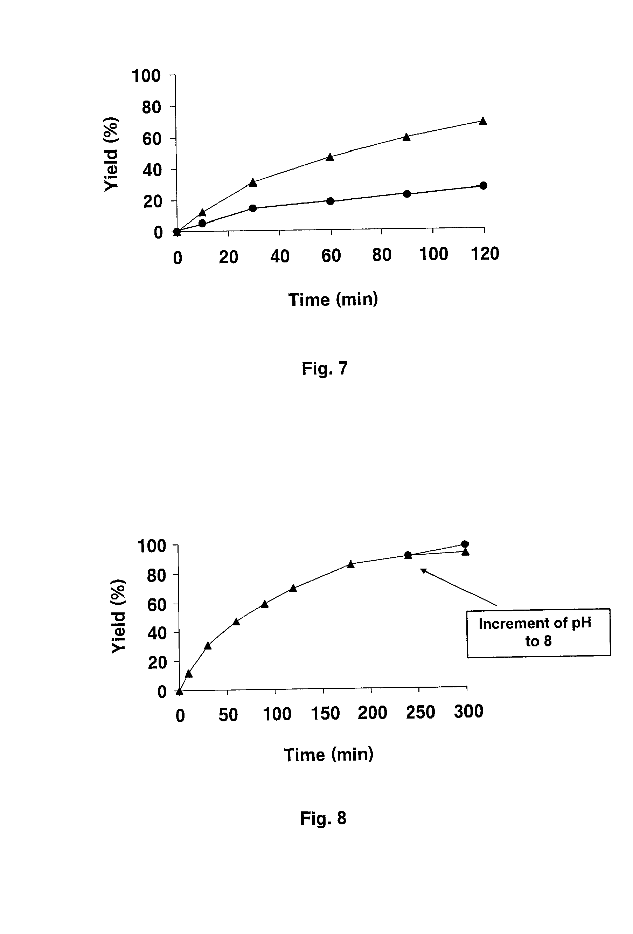

This activity significantly decreased in the presence of 100mM glutaric acid (see Fig. 7). Therefore the advantages of using pure α-ketoadipyl 7ACA to prevent this strong inhibition are clear.

The use of a higher pH value (pH 8) in the last step of the reaction permits the transformation of more than 98% of α-ketoadipyl 7ACA to 7 ACA as shown in Fig. 8.

### Example 6: Production of 7ACA from cephalosporin C in one reactor containing co-immobilised DAAO and catalase plus glutaryl acylase.

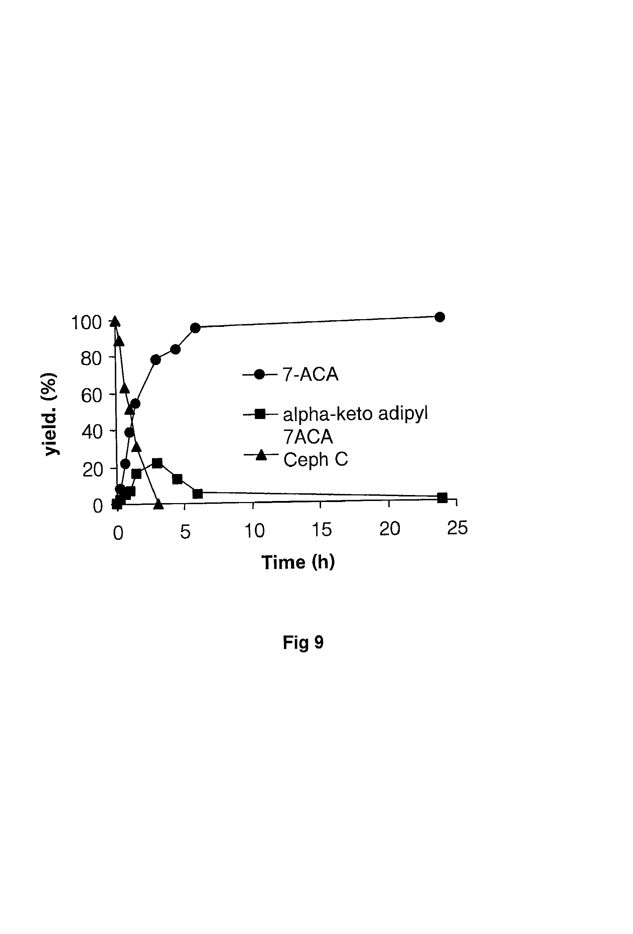

100 ml of Cephalosporin C (25 mM) was dissolved at pH 7.5 in 100 mM potassium phosphate at 22°C, 1 g of coimmobilised derivative prepared as described in example 3, and 5 g of glutaryl acylase were added to the reaction mixture under the bubbling of oxygen. Full transformation from Cephalosporin to 7ACA was achieved as shown in Fig. 9.

The invention is not limited to the embodiments which are hereinbefore described in detail.

References
1. Dey, E.S., Miller, J.R., Kovacevic S. & Mosbach, K. (1990) Characterization of D-amino acid oxidase with high activity against cephalosporin C from the yeast Trigonopsis variabilis *in Bioch. Mol. Biol. Int*. **20,** 1169-1178.
2. Dey, E,S., Flygare, S. & Mosbach, K. (1991) Stabilization of D-amino acid oxidase from yeast Trigonopsis variabilis used for production of glutarayl-7-aminocephalosporanic acid from Cephalosporin C *in Appl. Biochem. Biotechnol.* **27,** 239-250.
3. Golini, P., Bianchi, D., Battistel, E., Cesti, P & Tassinari, R. (1995) Immobilization of D-amino acid oxidase from different yeast: characterization and application in the deamination of cephalosporin C *in Enzyme Microb. Technol.* **17,** 324-329.
4. Nikolow, A. & Danielsson, B. (1994) Enzymatic transformation of cephalosporin C to 7ACA. Part II: single step procedure using a co-immobilized enzyme system *in Enzyme Microb. Technol.* **16.** 1037-1041.
5. Hernández-Justiz, O., Fernández-Lafuente, R., Guisán, J.M.; Negri, P., Pagani, G., Pregnolato. M. & Terreni, M. 1997. "One pot chemoenzymatic synthesis of 3'functionalized cephalosporins (cephazolin) by three consecutive biotransformations in fully aqueous medium" *J. Org, Chem.* **62,** 9099-9106.
6. Fernández-Lafuente, R., Rodríguez, V. & Guisán, J.M. 1998. "The coimmobilization of D-aminoacid oxidase and catalase enables the quantitative transformation of D-amino acids (phenylalanine) into alpha-ceto acids (phenylpyruvic acid). *Enzyme Microb. Technol.* 23, 28-33.
7. Blanco, R. M. Calvete, J.J. y Guisán, J. M. "Immobilization-stabilization of enzymes. Variables that control the intensity of the Trypsin (amine)-agarose (aldehyde) multipoint attachment." Enzyme Microbial Technology. Vol. 11, 1988, 353-359.
8. Blanco, R. M. y Guisán, J. M. "Stabilization of enzymes by mltipoint covalent attachment to agarose-aldehyde gels. Borohydre reduction of trypsin-agarose derivatives"Enzyme Microbial Technology. Vol. 11, 1988, 360-366.
9. Guisán, J. M. "Agarose-Aldehyde gels as supports for immobilization-stabilization of enzymes".Enzyme Microb. Technol. Vol. 10, 1988. 375-382.
10. Álvaro, G., Fernández-Lafuente, R., Blanco, R.M. & Guisán, J.M. 1990. "Immobilization-stabilization of penicillin G acylase from *Escherichia coli".* Applied Biochem. and Biotechnol. 26, 186-195.
11. Guisán, J.M., Bastida, A, Cuesta, C., Fernández-Lafuente, R. & Rosell, C.M. 1991. "Immobilisation-stabilisation of chymotrypsin by covalent attachment to aldehyde agarose gels" Biotechnol. Bioeng. 39, 75-84.
12. Guisán. J.M., Álvaro, G., Fernández-Lafuente, R., Rosell, C.M., García-López, J.L. & Tagliatti. A. 1993 "Stabilization of an heterodimeric enzyme by multipoint covalent immobilisation: Penicillin G acylase from *Kluyvera citrophila"* Biotechnol. Bioeng. 42, 455-464.
13. Mateo,C., Abian, O, Fernandez-Lafuente, R., & Guisán, J.M 2000 "Increase in conformational stability of enzymes immobilized on epoxy-activated supports by favoring additional multipoint covalent attachment" *Enzyme Microb. Technol.* 26, 509-515.
14. Fernández-Lafuente, R, Hernández-Jústiz, O., Mateo, C., Fernández-Lorente, G., Terreni, M., Alonso, J., Garcia-López, J.L., Moreno, M.A., Guisán, J.M 2001"Biotransformations catalyzed by multimeric enzymes: stabilization of tetrameric ampicillin acylase permits the optimization of ampicillin synthesis under dissociation conditions" *Biomocromolecules. 2, 95-104*

## Claims

1. A process for the preparation of cephalosporin acid derivatives comprising the step of enzymatically converting cephalosporin C into 7 cephalosporin acid derivatives in the presence of two biocatalysts, wherein one biocatalyst comprises co-immobilised D-amino acid oxidase and a catalase enzyme, and the other biocatalyst comprises immobilised glutaryl acylase.

2. A process as claimed in claim 1 wherein the reaction is carried out with the co-immobilised DAAO and catalase and the immobilised glutaryl acylase present in a single reactor.

3. A process as claimed in claim 1 or 2 wherein the reaction is carried out in a single basket reactor or other reactor type wherein the co-immobilised DAAO and catalase are physically separated from the immobilised glutaryl acylase.

4. A process as claimed in any of claims 1 to 3 wherein the three enzymes are present in one biocatalyst system comprising co-immobilised DAAO, catalase and glutaryl acylase enzymes.

5. A process as claimed in any of claims 1 to 4 wherein the or each biocatalyst system is immobilised on highly activated aldehyde or epoxy supports.

6. A process as claimed in any preceding claim wherein the or each biocatalyst system is immobilised on highly activated glutaraldehyde or glyoxyl supports.

7. A process as claimed in claim 1 or 2 wherein the D-amino acid oxidase and catalase enzymes are co-immobilised on highly activated glyoxyl or epoxy supports.

8. A process as claimed in any of claims 5 to 7 wherein the supports have large internal surfaces to permit intense multipoint and multi-subunit covalent attachment.

9. A process as claimed in claim 8 wherein the supports are selected from any one or more of agarose gels, silica porous beads, porous glass, epoxy acrylic resins or cellulose.

10. A process as claimed in any preceding claim wherein D-amino acid oxidase is obtained from one or more of *Rhodotorula gracilis* or *Trigonopsis variabilis.*

11. A process as claimed in any preceding claim wherein the catalase enzyme is obtained from one or more of mammalian liver, *Thermus sp*, *Bacillus sterothermophillus, Thermotoga sp, Micrococcus lysodeikticus* or *Aspergillus niger*.

12. A process as claimed in any preceding claim wherein glutaryl acylase is obtained from *Acinetobacter sp.*

13. Cephalosporin acid derivatives whenever produced by a process as claimed in any of claims 1 to 12.

14. Ketoadipyl 7-ACA whenever produced by a process as claimed in any of claims 1 to 12.
